# EUROPÄISCHE PATENTSCHRIFT

(11) **EP 0 835 245 B1**
(45) Veröffentlichungstag und Bekanntmachung des Hinweises auf die Patenterteilung: **26.10.2005**
(21) Anmeldenummer: 96922003.7
(22) Anmeldetag: 17.06.1996
(51) Int. Cl.: C07D 213/61, C07D 401/06, C07D 417/06, A01N 43/78, A01N 43/50, A01N 43/40

(54) **SUBSTITUIERTE N-METHYLENTHIOHARNSTOFFE ALS SCHÄDLINGSBEKÄMPFUNGSMITTEL**
SUBSTITUTED N-METHYLENETHIOUREAS AS PESTICIDES
N-METHYLENETHIO-UREES SUBSTITUEES UTILISEES COMME PARASITICIDES

(30) Priorität: 29.06.1995 DE 19523658
(43) Veröffentlichungstag der Anmeldung: 15.04.1998
(73) Patentinhaber: Bayer CropScience AG, 40789 Monheim (DE)
(72) Erfinder: KANELLAKOPULOS, Johannes, D-41542 Dormagen (DE); FINDEISEN, Kurt, D-51375 Leverkusen (DE); LINKER, Karl-Heinz, D-51377 Leverkusen (DE); ERDELEN, Christoph, D-42799 Leichlingen (DE); TURBERG, Andreas, D-40699 Erkrath (DE); MENCKE, Norbert, D-51381 Leverkusen (DE)
(86) Internationale Anmeldenummer: PCT/EP1996/002602
(87) Internationale Veröffentlichungsnummer: WO 1997/001537

(56) Entgegenhaltungen:
- EP-A- 0 161 841
- EP-A- 0 268 915
- EP-A- 0 306 696
- EP-A- 0 639 569
- SYNTHETIC COMMUNICATIONS, Bd. 26, Nr. 7, 1996, NEW YORK, Seiten 1423-1429, XP000603788 L.A. REITER ET AL.: "Synthesis of mono N-substituted guanylthioureas"
- BULLETIN DES SOCIETES CHIMIQUES BELGES, Bd. 87, Nr. 3, 1978, OXFORD GB, Seiten 223-228, XP002015264 B.S. PEDERSEN ET AL.: "Studies on organophosphorus compounds XX. Syntheses of thioketones." in der Anmeldung erwähnt

## Beschreibung

Die vorliegende Erfindung betrifft neue substituierte N-Methylenthioharnstoffe, ein Verfahren zu ihrer Herstellung und ihre Verwendung in Schädlingsbekämpfungsmitteln, insbesondere als Insektizide.

Im Stand der Technik werden N-substituierte Amidino(thio)harnstoffe und deren Anwendung als Pharmazeutika beschrieben, die sich durch die Anwesenheit einer (Thio)Carbonyl-Gruppe im Molekül von den N-Methylenthioharnstoffen der vorliegenden Anmeldung unterscheiden (BE 894 172; US 4 701 457).

Darüber hinaus werden in der JP 53 108 970 Guanidin-Derivate beschrieben die sich ebenfalls im pharmazeutischen Bereich zur Regulierung der Sekretion von Magensäuren einsetzen lassen. Eine insektizide Wirkung der Verbindungen aus dem nächsten Stand der Technik ist nicht bekannt.

In EP 0 306 696 werden insektizid wirksame N-Pyridylmethyl-N'-cyano-guanidine beschrieben, die jedoch keine Thioharnstoffstruktur besitzen.

Ferner werden in EP 0 639 569 insektizid wirksame heterocyclische Verbindungen beschrieben, die sich von den erfindungsgemäßen Verbindungen im Heterocyclenteil strukturell unterscheiden.

Es wurden nun die neuen substituierten N-Methylenthioharnstoffe der allgemeinen Formel (I) gefunden, 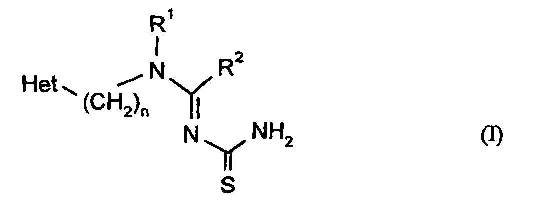 in welcher
- n: für eine Zahl 0, 1 oder 2 steht,
- Het: für eine fünf- bzw. sechsgliedrige heterocyclische Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl steht, welche durch Fluor, Chlor, Brom, Iod, Cyano, Nitro, C₁-C₄-Alkyl (welches gegebenenfalls durch Fluor und/oder Chlor substituiert ist), C₂-C₄-Alkenyl (welches gegebenenfalls durch Fluor und/oder Chlor substituiert ist), C₂-C₄-Alkinyl, C₁-C₄-Alkoxy (welches gegebenenfalls durch Fluor und/oder Chlor substituiert ist), C₃-C₄-Alkenyloxy (welches gegebenenfalls durch Fluor und/oder Chlor substituiert ist), C₃-C₄-Alkinyloxy, C₁-C₄-Alkylthio (welches gegebenenfalls durch Fluor und/oder Chlor substituiert ist), C₃-C₄-Alkenylthio (welches gegebenenfalls durch Fluor und/oder Chlor substituiert ist), C₃-C₄-Alkinylthio, C₁-C₄-Alkylsulfinyl (welches gegebenenfalls durch Fluor und/oder Chlor substituiert ist), C₁-C₄-Alkylsulfonyl (welches gegebenenfalls durch Fluor und/oder Chlor substituiert ist), Amino, C₁-C₄-Alkylamino, Di-(C₁-C₄-alkyl)-amino, Phenyl, Phenoxy, Phenylthio, Phenylamino, Benzyl, Formylamino, C₁-C₄-Alkyl, Carbonylamino, Formyl, Carbamoyl, C₁-C₄-Alkylcarbonyl und/oder C₁-C₄-Alkoxy-carbonyl substituiert ist und
- R¹: für Wasserstoff oder C₁-C₆-Alkyl steht,
- R²: für Wasserstoff, C₁-C₆-Alkyl oder eine der Gruppen NR³R⁴, SR⁵ oder OR⁵ steht,
R³ für Wasserstoff oder C₁-C₆-Alkyl steht,
R⁴ für Wasserstoff oder C₁-C₆-Alkyl steht,
R⁵ für C₁-C₆-Alkyl steht, oder
- R¹ und R³ bzw. R¹ und R⁵: gemeinsam eine gegebenenfalls substituierte Ethylen- oder Propylenbrücke bilden, die gegebenenfalls durch 1 weiteres Sauerstoff-, Stickstoff- und/oder Schwefelatome unterbrochen und gegebenenfalls durch folgende Substituenten substituiert sein kann:
Fluor, Chlor, Brom, Cyano, C₁-C₄-Alkyl, C₁-C₄-Alkoxy, C₂-C₄-Alkenyl, C₂-C₄-Alkinyl.

Weiterhin wurde gefunden, dass man die neuen substituierten N-Methylenthioharnstoffe der allgemeinen Formel (I) erhält, wenn man die entsprechenden N'-Aralkyl-N-cyanoamidine der allgemeinen Formel (II) 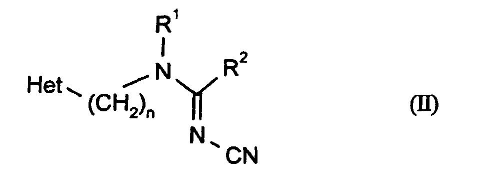 in welcher
Het, n, R¹ und R² die oben genannte Bedeutung haben,
mit Lawessons's Reagenz gegebenenfalls in Gegenwart eines Verdünnungsmittels, gegebenenfalls unter Feuchtigkeitsausschluss umsetzt.

Überraschenderweise zeichnen sich die neuen substituierten N-Methylenthioharnstoffe der allgemeinen Formel (I) in überraschender Weise durch eine hohe Wirksamkeit als Insektizide aus.

Die Erfindung betrifft vorzugsweise Verbindungen der Formel (I), in welcher
- n: für eine Zahl 0, 1 oder 2 steht,
- Het: für eine fünf- bzw. sechsgliedrige heterocyclische Gruppierung aus der Reihe Pyrazolyl, 1,2,4-Triazolyl, Oxazolyl, Isoxazolyl, Thiazolyl, Isothiazolyl, 1,2,5-Thiadiazolyl, Pyridyl, Pyrazinyl und Pyrimidinyl steht, welche durch Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist) oder C₁-C₂-Alkylsulfonyl (welches gegebenenfalls durch Fluor und/oder Chlor substituiert ist) substituiert ist,
- R¹: für Wasserstoff oder C₁-C₄-Alkyl steht,
- R²: für Wasserstoff, C₁-C₄-Alkyl oder eine der Gruppen NR³R⁴, SR⁵ oder OR⁵ steht,
R³ und R⁴ jeweils für Wasserstoff oder C₁-C₄-Alkyl stehen,
R⁵ für C₁-C₄-Alkyl steht, oder
- R¹ und R³ bzw. R¹ und R⁵: gemeinsam eine gegebenenfalls substituierte Ethylen- oder Propylenbrücke bilden können, die gegebenenfalls durch 1 weiteres Sauerstoff-, Stickstoff- oder Schwefelatom unterbrochen und gegebenenfalls durch folgende Substituenten substituiert sein kann:
Fluor, Chlor, Brom, Cyano, Methyl, Ethyl, n-, i-Propyl, n-, i-, s-, t-Butyl.

Ganz besonders bevorzugt sind Verbindungen der Formel (I), in welcher
- n: für die Zahl 1 steht,
- Het: für durch Fluor oder Chlor substituiertes Pyridyl oder für durch Brom oder Chlor substituiertes Thiazolyl steht,
- R¹: für Wasserstoff, Methyl oder Ethyl steht,
- R²: für Wasserstoff, Methyl oder Ethyl oder eine der Gruppen NR³R⁴, SR⁵ oder OR⁵ steht,
R³ und R⁴ jeweils für Wasserstoff, Methyl oder Ethyl stehen,
R⁵ für Methyl oder Ethyl steht oder
- R¹ und R³ bzw. R¹ und R⁵: zusammen eine -CH₂-CH₂-Gruppe bilden.

Verwendet man beispielsweise das erfindungsgemäße N-(2-Chlor-5-pyridylmethyl) als Ausgangsstoff, so kann die entsprechende Reaktion durch das folgende Formelschema wiedergegeben werden:

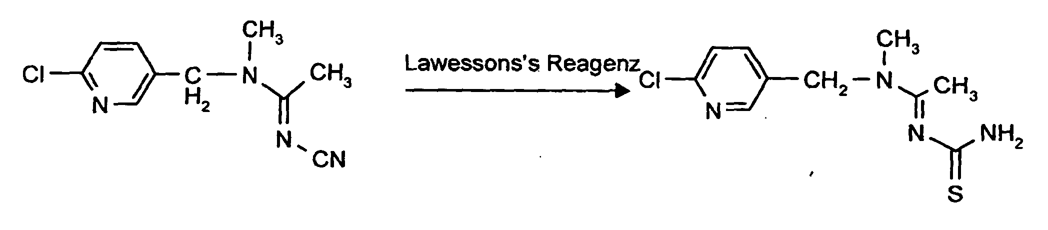

Die bei dem erfindungsgemäßen Verfahren als Ausgangsstoffe zu verwendenden N-Cyanoverbindungen sind durch die Formel (II) allgemein definiert. In dieser Formel (II) haben Het, n, R¹ und R² vorzugsweise diejenige Bedeutung, die bereits im Zusammenhang mit der Beschreibung der erfindungsgemäßen Stoffe der Formel (I) vorzugsweise für Het, n, R¹ und R² genannt wurden.

Die Verbindungen der Formel (II) sind bekannt und/oder lassen sich nach bekannten Methoden herstellen.

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen alle für die Umsetzung üblichen inerten organischen Lösungsmittel in Frage.

Beispiele für solche Verdünnungsmittel umfassen Ether, wie Diethylether, Di-isopropylether, Methyl-tert.-butylether, Dimethoxyethan, Dioxan, Tetrahydrofuran, Säureamide, wie Dimethylformamid, Dimethylacetamid, N-Methylpyrrolidon und Sulfoxide, wie Dimethylsulfoxid.

Das erfindungsgemäße Verfahren zur Herstellung der neuen Verbindungen der Formel (I) wird vorzugsweise unter Verwendung von Lawessons's Reagenz [2,4-Bis-(4-methoxyphenyl)-1,3-dithia-2,4-diphosphetan-2,4-disulfid; Bull. Soc. Chim. Belg. 87, 223, 229, 299, 525 (1978)] durchgeführt.

Die Reaktionstemperaturen können bei dem erfindungsgemäßen Verfahren in einem größeren Bereich variiert werden. Im Allgemeinen arbeitet man bei Temperaturen zwischen 0°C und 50°C, vorzugsweise bei Temperaturen zwischen 15°C und 30°C.

Das erfindungsgemäße Verfahren wird im Allgemeinen unter Normaldruck durchgeführt. Es ist jedoch auch möglich, unter erhöhtem oder vermindertem Druck zu arbeiten.

Zur Durchführung des Verfahrens zur Herstellung der neuen Verbindungen der Formel (I) werden die jeweils benötigten Ausgangsstoffe im Allgemeinen in angenähert äquimolaren Mengen eingesetzt. Es ist jedoch auch möglich, eine der jeweils eingesetzten Komponenten in einem größeren Überschuss zu verwenden. Die Reaktionen werden im Allgemeinen in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die Herstellung der erfindungsgemäßen Verbindungen geht aus den nachfolgenden Beispielen hervor.

### Herstellungsbeispiel

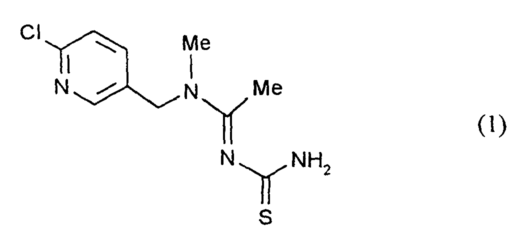

Ein Gemisch von 3,0 g (13,4 mmol) "Nl-25" und 5,25 g Lawesson's Reagenz in 50 ml THF wird 12 Stunden bei 50°C unter Feuchtigkeitsausschluß gerührt. Das Lösungsmittel wird unter vermindertem Druck abdestilliert, der Rückstand mit 25 ml 1M-Natriumcarbonat-Lösung versetzt und das Gemisch mit Essigsäureethylester extrahiert. Chromatographie (Kieselgel, Essigsäureethylester/Cyclohexan 1:1) des nach Abtrennen, Trocknen und Abdestillieren der organischen Phase erhaltenen Rückstands liefert 1,1 g (31 %) Produkt 1-Thiacarbamoyl-3-N-methyl-3-N-(6-chlopyridyl-3-yl)methylacetamidin mit dem log P = 1,58 (sauer).

Analog dem Herstellungsbeispiel können weitere Verbindungen der Formel (I) hergestellt werden wie sie in folgender Tabelle aufgeführt werden:

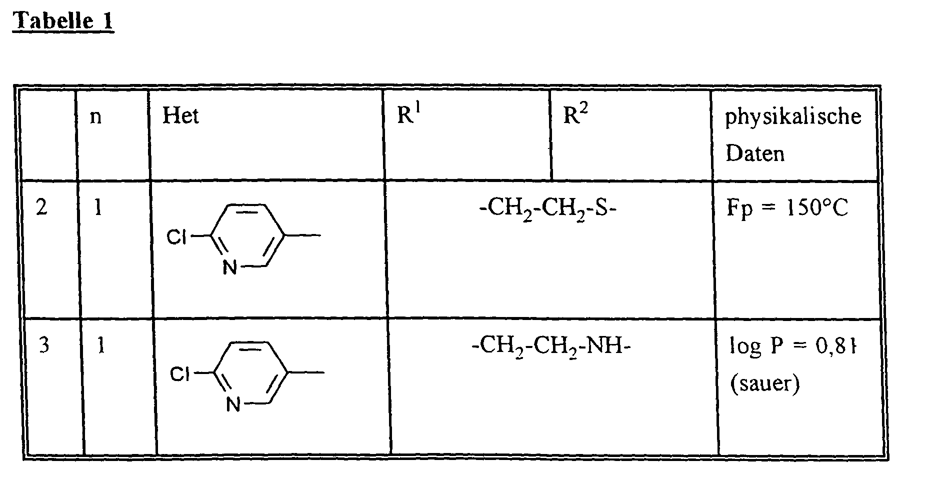

Die Wirkstoffe eignen sich zur Bekämpfung von tierischen Schädlingen, vorzugsweise Arthropoden insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria. Aus der Ordnung der Dermaptera z.B. Forficula auricularia.
Aus der Ordnung der Isoptera z.B. Reticulitermes spp.
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.

Die erfindungsgemäßen Wirkstoffe der Formel (I) zeichnen sich durch eine hervorragende insektizide Wirksamkeit aus. Sie zeigen insbesondere beim Einsatz als Blattinsektizide und Bodeninsektizide eine hervorragende Wirkung gegen Käferlarven, wie z.B. Phaedon cochleariae und Diabrotica balteata, Blattläuse, wie z.B. Myzus persicae und Zikaden, wie z.B. Nephotettix cincticeps.

Darüber hinaus besitzen die erfindungsgemäßen Wirkstoffe der Formel (I) auch eine hervorragende wurzelsystemische Wirkung.

Die neuen Verbindungen sind also besonders gut für einen Einsatz zur Bekämpfung von Blattinsekten und Bodeninsekten und als wurzelsystemische Wirkstoffe geeignet.

Ferner besitzen einige der neuen Verbindungen auch fungizide Wirkung gegen Pyricularia oryzae und Pellicularia sasakii.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.N. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgut, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die biologische Wirksamkeit der erfindungsgemäßen Verbindungen soll anhand der folgenden Beispiele erläutert werden.

### Beispiel

### Phaedon-Larven-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik:

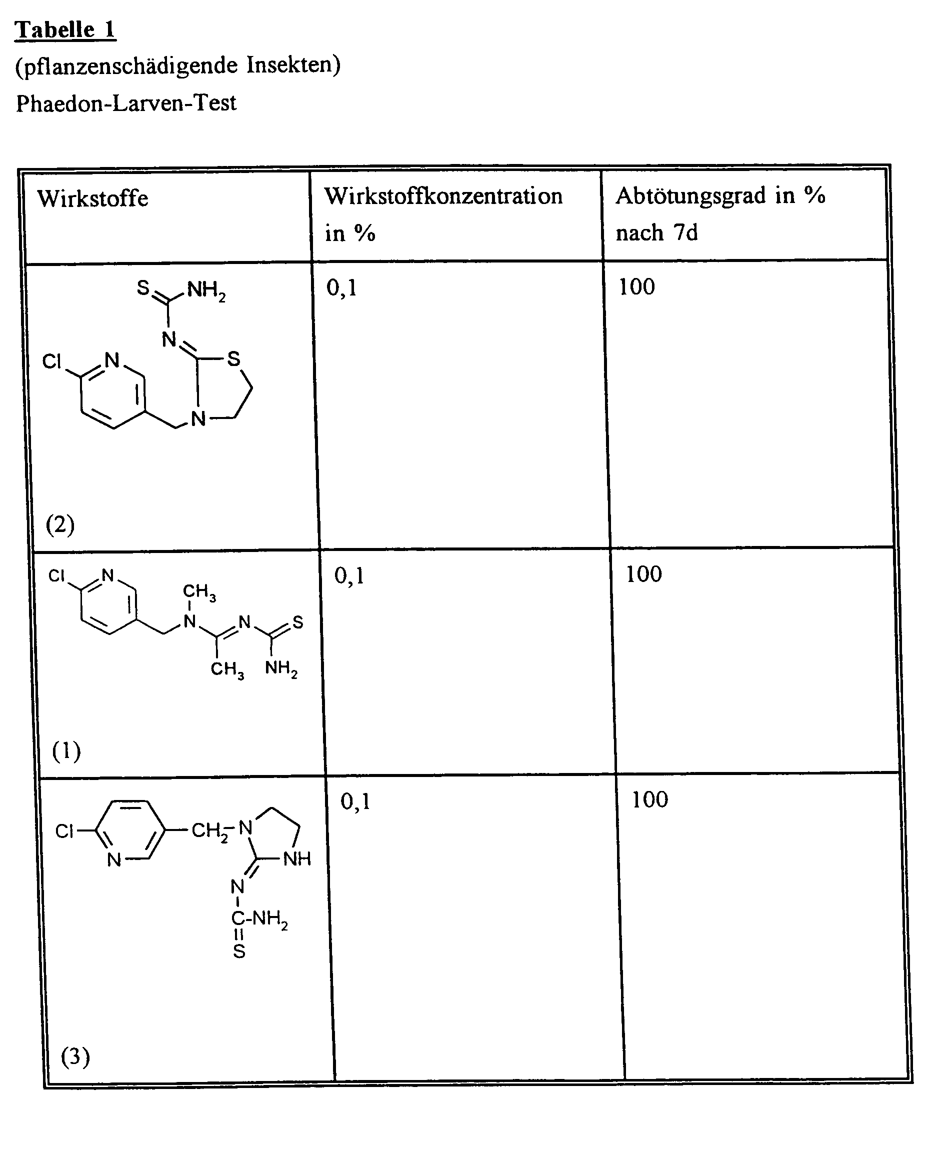

### Beispiel

### Plutella-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik:

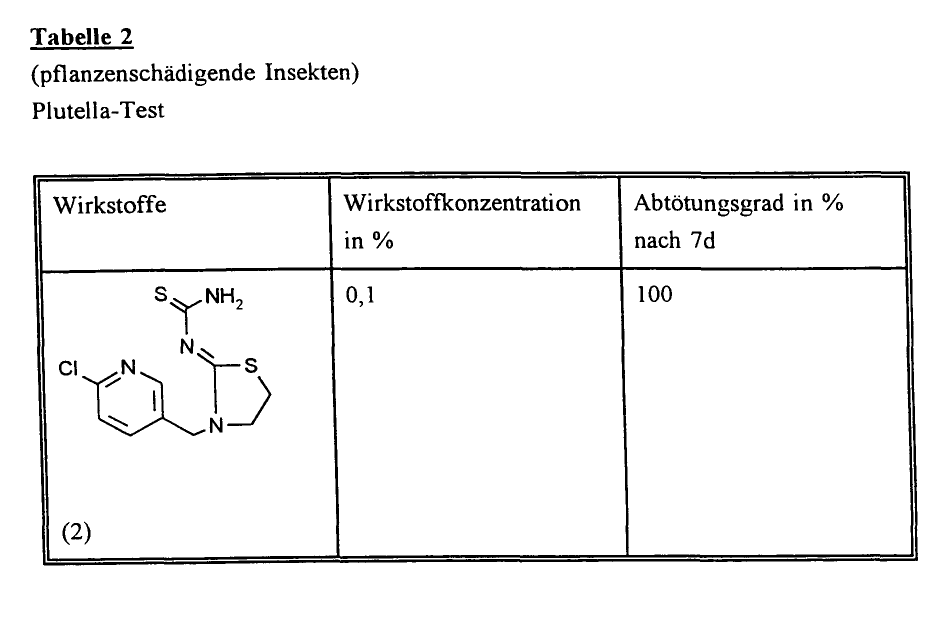

### Beispiel

### Spodoptera-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik:

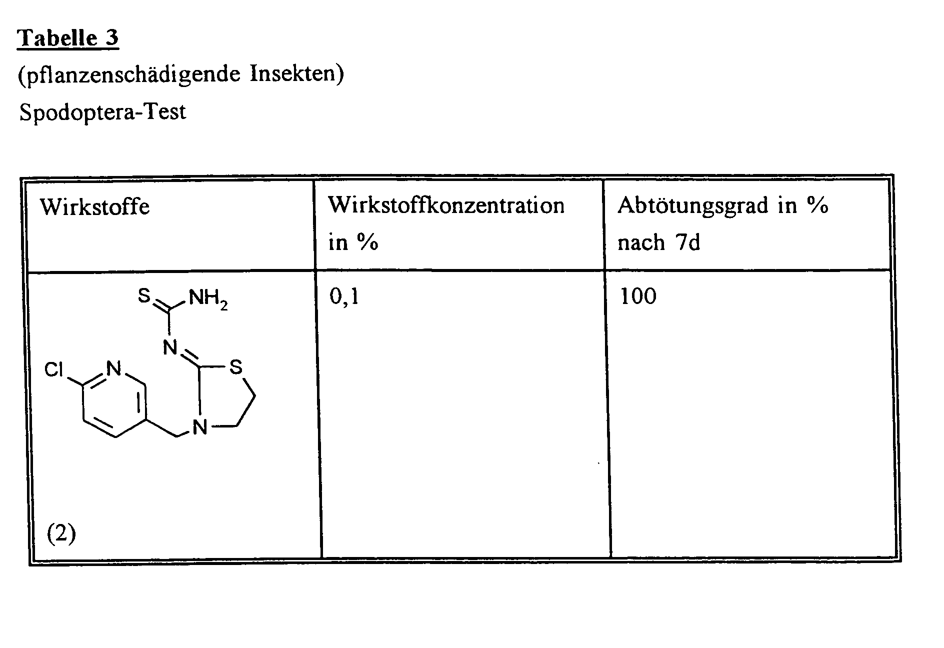

### Beispiel

### Nephotettix-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryzae sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z.B. die folgenden Verbindungen der Herstellungsbeispiele überlegene Wirksamkeit gegenüber dem Stand der Technik:

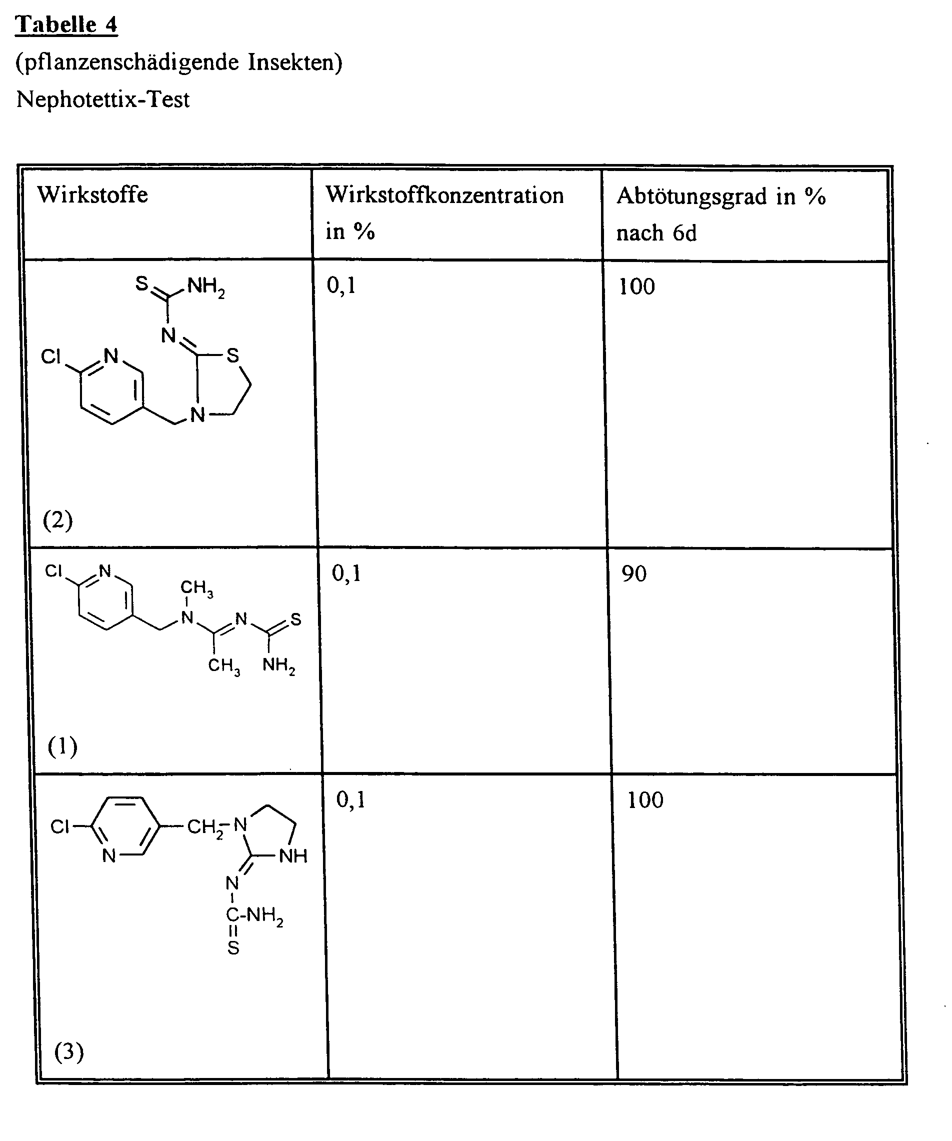

### Beispiel

### Myzus-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik:

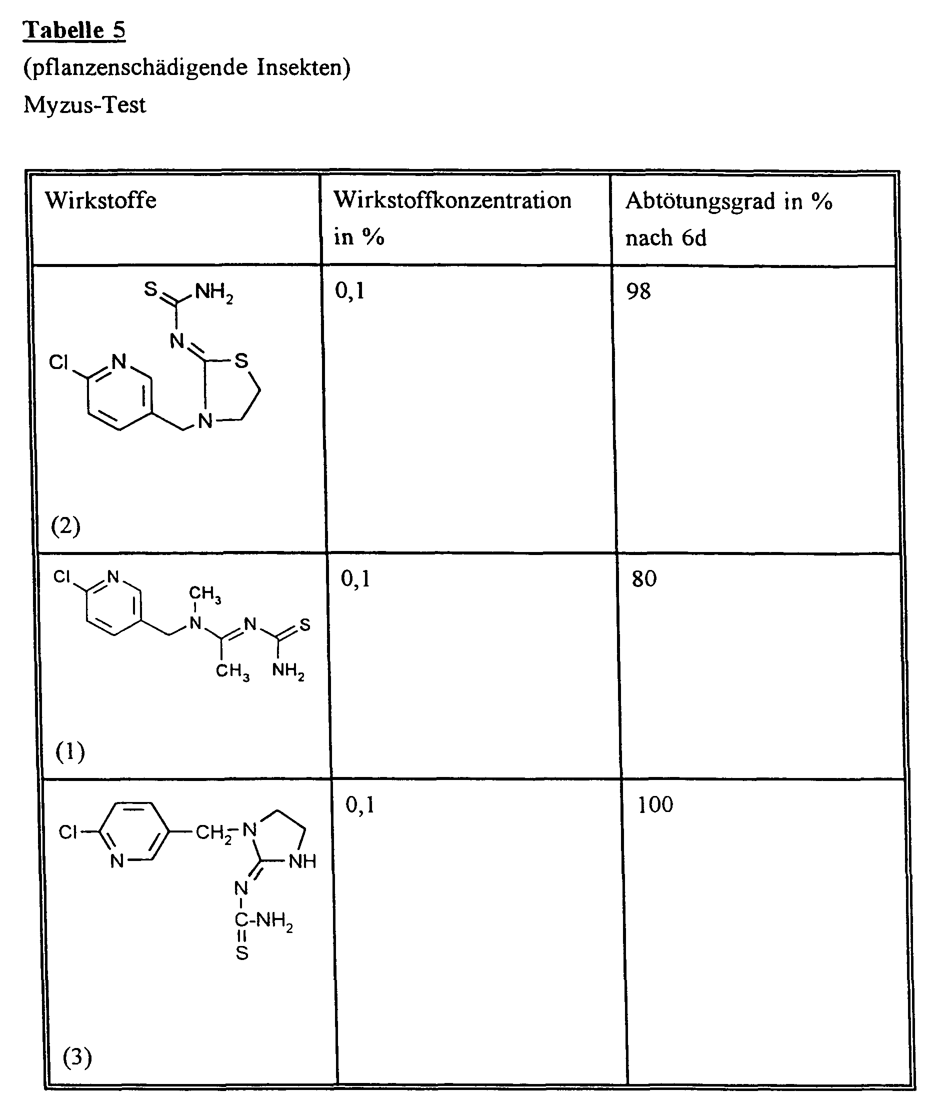

### Beispiel

### Blowfly-Larven-Test / Entwicklungshemmende Wirkung

| | |
|---|---|
| Testtiere | Lucilia cuprina-Larven |
| Lösungsmittel | 35 Gewichtsteile Ethylenglykolmonomethylether |
| | 35 Gewichtsteile Nonylphenolpolyglykolether |

Zwecks Herstellung einer geeigneten Formulierung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die jeweils gewünschte Konzentration.

Etwa 20 Lucilia cuprina-Larven werden in ein Teströhrchen gebracht, welches ca. 1 cm³ Pferdefleisch und 0,5 ml der zu testenden Wirkstoffzubereitung enthält. Nach 24 und 48 Stunden wird die Wirksamkeit der Wirkstoffzubereitung ermittelt. Die Teströhrchen werden in Becher mit Sand-bedecktem Boden überführt. Nach weiteren 2 Tagen werden die Teströhrchen entfernt und die Puppen ausgezählt.

Die Wirkung der Wirkstoffzubereitung wird nach der Zahl der geschlüpften Fliegen nach 1,5-facher Entwicklungsdauer und unbehandelter Kontrolle beurteilt. Dabei bedeutet 100 %, daß keine Fliegen geschlüpft sind; 0 % bedeutet, daß alle Fliegen normal geschlüpft sind.

Bei diesem Test zeigen z.B. die folgenden Verbindungen der Herstellungsbeispiele überlegene Wirkung gegenüber dem Stand der Technik:

**Tabelle 6**

| Blowfly-Larven-Test | | |
|---|---|---|
| | Konzentration | % Wirkung/Abtötung |
| erfindungsgemäß | Lucillia cuprina | |
| (2) | 1000 | 100 |
| (3) | 1000 | 100 |

### Beispiel

### Schabentest

| | |
|---|---|
| Testtiere | Periplaneta americana |
| Lösungsmittel | 35 Gewichtsteile Ethylenglykolmonomethylether |
| Emulgator | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (Ø 9,5 cm) pipettiert, die sich in Petrischalen entsprechender Größe befinden. Nach Trocknung der Filterscheiben werden 5 Testtiere P. americana überführt und abgedeckt.

Nach 3 Tagen wird die Wirksamkeit der Wirkstoffzubereitung bestimmt. Dabei bedeutet 100 %, daß alle Schaben abgetötet wurden; 0 % bedeutet, daß keine Schaben abgetötet wurden.

In diesem Test zeigen z.B. die folgenden Verbindungen der Herstellungsbeispiele überlegene Wirkung gegenüber dem Stand der Technik.

**Tabelle 7**

| Schabentest | | |
|---|---|---|
| | Konzentration | % Wirkung/Abtötung |
| erfindungsgemäß | Periplaneta americana | |
| (3) | 1000 | 100 |

## Patentansprüche

1. Substituierte N-Methylenthioharnstoffe der allgemeinen Formel (I) 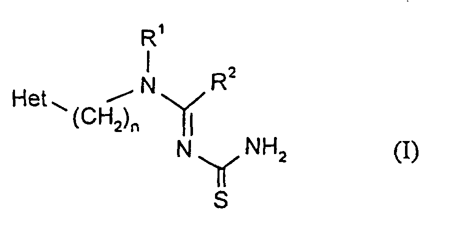 in welcher
n für eine Zahl 0, 1 oder 2 steht,
Het für eine fünf- bzw. sechsgliedrige heterocyclische Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl steht, welche durch Fluor, Chlor, Brom, Iod, Cyano, Nitro, C₁-C₄-Alkyl (welches gegebenenfalls durch Fluor und/oder Chlor substituiert ist), C₂-C₄-Alkenyl (welches gegebenenfalls durch Fluor und/oder Chlor substituiert ist), C₂-C₄-Alkinyl, C₁-C₄-Alkoxy (welches gegebenenfalls durch Fluor und/oder Chlor substituiert ist), C₃-C₄-Alkenyloxy (welches gegebenenfalls durch Fluor und/oder Chlor substituiert ist), C₃-C₄-Alkinyloxy, C₁-C₄-Alkylthio (welches gegebenenfalls durch Fluor und/oder Chlor substituiert ist), C₃-C₄-Alkenylthio (welches gegebenenfalls durch Fluor und/oder Chlor substituiert ist), C₃-C₄-Alkinylthio, C₁-C₄-Alkylsulfinyl (welches gegebenenfalls durch Fluor und/oder Chlor substituiert ist), C₁-C₄-Alkylsulfonyl (welches gegebenenfalls durch Fluor und/oder Chlor substituiert ist), Amino, C₁-C₄-Alkylamino, Di-(C₁-C₄-alkyl)-amino, Phenyl, Phenoxy, Phenylthio, Phenylamino, Benzyl, Formylamino, C₁-C₄-Alkyl, Carbonylamino, Formyl, Carbamoyl, C₁-C₄-Alkyl-carbonyl und/oder C₁-C₄-Alkoxy-carbonyl substituiert ist und
R¹ für Wasserstoff oder C₁-C₆-Alkyl steht,
R² für Wasserstoff, C₁-C₆-Alkyl oder eine der Gruppen NR³R⁴, SR⁵ oder OR⁵ steht,
R³ für Wasserstoff oder C₁-C₆-Alkyl steht,
R⁴ für Wasserstoff oder C₁-C₆-Alkyl steht,
R⁵ für C₁-C₆-Alkyl steht, oder
R¹ und R³ bzw. R¹ und R⁵ gemeinsam eine gegebenenfalls substituierte Ethylen- oder Propylenbrücke bilden, die gegebenenfalls durch 1 weiteres Sauerstoff-, Stickstoff- und/oder Schwefelatome unterbrochen und gegebenenfalls durch folgende Substituenten substituiert sein kann:
Fluor, Chlor, Brom, Cyano, C₁-C₄-Alkyl, C₁-C₄-Alkoxy, C₂-C₄-Alkenyl, C₂-C₄-Alkinyl.

2. Substituierte N-Methylenthioharnstoffe der allgemeinen Formel (I) gemäß Anspruch 1, **dadurch gekennzeichnet, daß**
n für eine Zahl 0, 1 oder 2 steht,
Het für eine fünf- bzw. sechsgliedrige heterocyclische Gruppierung aus der Reihe Pyrazolyl, 1,2,4-Triazolyl, Oxazolyl, Isoxazolyl, Thiazolyl, Isothiazolyl, 1,2,5-Thiadiazolyl, Pyridyl, Pyrazinyl und Pyrimidinyl steht, welche durch Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist) oder C₁-C₂-Alkylsulfonyl (welches gegebenenfalls durch Fluor und/oder Chlor substituiert ist) substituiert ist,
R¹ für Wasserstoff oder C₁-C₄-Alkyl steht,
R² für Wasserstoff, C₁-C₄-Alkyl oder eine der Gruppen NR³R⁴, SR⁵ oder OR⁵ steht,
R³ und R⁴ jeweils für Wasserstoff oder C₁-C₄-Alkyl stehen,
R⁵ für C₁-C₄-Alkyl steht, oder
R¹ und R³ bzw. R¹ und R⁵ gemeinsam eine gegebenenfalls substituierte Ethylen- oder Propylenbrücke bilden können, die gegebenenfalls durch 1 weiteres Sauerstoff-, Stickstoff- oder Schwefelatom unterbrochen und gegebenenfalls durch folgende Substituenten substituiert sein kann:
Fluor, Chlor, Brom, Cyano, Methyl, Ethyl, n-, i-Propyl, n-, i-, s-, t-Butyl.

3. Substituierte N-Methylenthioharnstoffe der allgemeinen Formel (I) gemäß Anspruch 1, **dadurch gekennzeichnet, daß**
n für die Zahl 1 steht,
Het für durch Fluor oder Chlor substituiertes Pyridyl oder für durch Brom oder Chlor substituiertes Thiazolyl steht,
R¹ für Wasserstoff, Methyl oder Ethyl steht,
R² für Wasserstoff, Methyl oder Ethyl oder eine der Gruppen NR³R⁴, SR⁵ oder OR⁵ steht,
R³ und R⁴ jeweils für Wasserstoff, Methyl oder Ethyl stehen,
R⁵ für Methyl oder Ethyl steht oder
R¹ und R³ bzw. R¹ und R⁵ zusammen eine -CH₂-CH₂-Gruppe bilden.

4. Verfahren zur Herstellung substituierter N-Methylenthioharnstoffe der allgemeinen Formel (I) 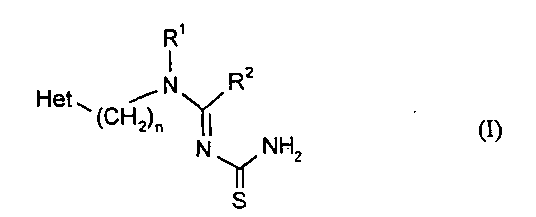 in welcher
n, Het, R¹ und R² die in Anspruch 1 genannten Bedeutungen haben,
**dadurch gekennzeichnet, daß** man die entsprechenden N'-Aralkyl-N-cyanoamidine der allgemeinen Formel (II) 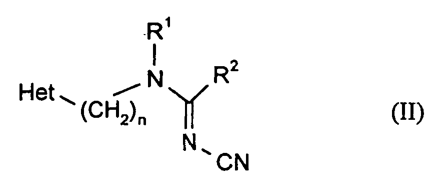 in welcher
Het, n, R¹ und R² die obengenannte Bedeutung haben,
mit Lawessons's Reagenz gegebenenfalls in Gegenwart eines Verdünnungsmittels, gegebenenfalls unter Feuchtigkeitsausschluß umsetzt.

5. Schädlingsbekämpfungsmittel, **gekennzeichnet durch** einen Gehalt an mindestens einem N-Methylenthioharnstoff der allgemeinen Formel (I) gemäß Anspruch 1.

6. Verfahren zur Bekämpfung von tierischen Schädlingen, insbesondere von Insekten, **dadurch gekennzeichnet, daß** man N-Methylenthioharnstoffe der allgemeinen Formel (I) gemäß Anspruch 1 auf tierische Schädlinge und/oder ihren Lebensraum einwirken läßt.

7. Verwendung von N-Methylenthioharnstoffen der allgemeinen Formel (I) gemäß Anspruch 1 zur Bekämpfung von tierischen Schädlingen, insbesondere von Insekten.

8. Insektizide Mittel, **gekennzeichnet durch** einen Gehalt an mindestens einem N-Methylenthioharnstoff der allgemeinen Formel (I) gemäß Anspruch 1.

9. Verfahren zur Herstellung von Mitteln gegen tierische Schädlinge, **dadurch gekennzeichnet, daß** man N-Methylenthioharnstoffe der allgemeinen Formel (I) gemäß Anspruch 1 mit Streckmitteln und/oder oberflächenaktiven Mitteln vermischt.

## Claims

1. Substituted N-methylenethioureas of the general formula (I) 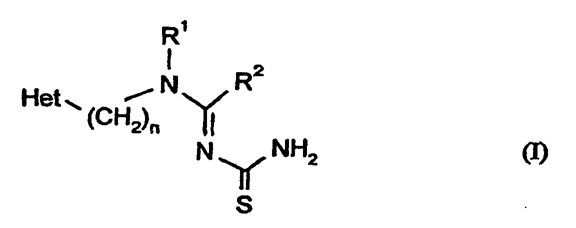 in which
n represents a number 0, 1 or 2,
Het represents a five- or six-membered heterocyclic group from the series consisting of furyl, thienyl, pyrrolyl, pyrazolyl, imidazolyl, 1,2,3- or 1,2,4-triazolyl, oxazolyl, isoxazolyl, 1,2,4- or 1,3,4-oxadiazolyl, thiazolyl, isothiazolyl, 1,2,3-, 1,2,4-, 1,2,5- or 1,3,4-thiadiazolyl, pyridyl, pyridazinyl, pyrimidinyl and pyrazinyl, which is substituted by fluorine, chlorine, bromine, iodine, cyano, nitro, C₁-C₄-alkyl (which is optionally substituted by fluorine and/or chlorine), C₂-C₄-alkenyl (which is optionally substituted by fluorine and/or chlorine), C₂-C₄-alkinyl, C₁-C₄-alkoxy (which is optionally substituted by fluorine and/or chlorine), C₃-C₄-alkenyloxy (which is C₃-C₄-alkenyloxy (which is optionally substituted by fluorine and/or chlorine), C₃-C₄-alkinyloxy, C₁-C₄-alkylthio (which is optionally substituted by fluorine and/or chlorine), C₃-C₄-alkenylthio (which is optionally substituted by fluorine and/or chlorine), C₃-C₄-alkinylthio, C₁-C₄-alkylsulphinyl (which is optionally substituted by fluorine and/or chlorine), C₁-C₄-alkylsulphonyl (which is substituted by fluorine and/or chlorine), amino, C₁-C₄-alkylamino, di-(C₁-C₄-alkyl)-amino, phenyl, phenoxy, phenylthio, phenylamino, benzyl, formylamino, C₁-C₄-alkyl, carbonylamino, formyl, carbamoyl, C₁-C₄-alkyl-carbonyl and/or C₁-C₄-alkoxy-carbonyl, and
R¹ represents hydrogen or C₁-C₆-alkyl,
R² represents hydrogen, C₁-C₆-alkyl or one of the groups NR³R⁴, SR⁵ or OR⁵,
R³ represents hydrogen or C₁-C₆-alkyl,
R⁴ represents hydrogen or C₁-C₆-alkyl,
R⁵ represents C₁-C₆-alkyl, or
R¹ and R³ or R¹ and R⁵ together form an optionally substituted ethylene or propylene bridge which can optionally be interrupted by 1 further oxygen, nitrogen and/or sulphur atoms and optionally substituted by the following substituents:
fluorine, chlorine, bromine, cyano, C₁-C₄-alkyl, C₁-C₄-alkoxy, C₂-C₄-alkenyl, C₂-C₄-alkinyl.

2. Substituted N-methylenethioureas of the general formula (I) according to Claim 1, **characterized in that**
n represents a number 0, 1 or 2,
Het represents a five- or six-membered heterocyclic group from the series consisting of pyrazolyl, 1,2,4-triazolyl, oxazolyl, isoxazolyl, thiazolyl, isothiazolyl, 1,2,5-thiadiazolyl, pyridyl, pyrazinyl and pyrimidinyl, which is substituted by fluorine, chlorine, bromine, cyano, nitro; C₁-C₂-alkyl (which is optionally substituted by fluorine and/or chlorine), C₁-C₂-alkoxy (which is optionally substituted by fluorine and/or chlorine), C₁-C₂-alkylthio (which is optionally substituted by fluorine and/or chlorine) or C₁-C₂-alkylsulphonyl (which is optionally substituted by fluorine and/or chlorine),
R¹ represents hydrogen or C₁-C₄-alkyl,
R² represents hydrogen, C₁-C₄-alkyl or one of the groups NR³R⁴, SR⁵ or OR⁵,
R³ and R⁴ in each case represent hydrogen or C₁-C₄-alkyl,
R⁵ represents C₁-C₄-alkyl, or
R¹ and R³ or R¹ and R⁵ together can form an optionally substituted ethylene or propylene bridge which can optionally be interrupted by 1 further oxygen, nitrogen or sulphur atom and optionally substituted by the following substituents:
fluorine, chlorine, bromine, cyano, methyl, ethyl, n-, i-propyl and n-, i-, s-, t-butyl.

3. Substituted N-methylenethioureas of the general formula (I) according to Claim 1, **characterized in that**
n represents the number 1,
Het represents pyridyl which is substituted by fluorine or chlorine or thiazolyl which is substituted by bromine or chlorine,
R¹ represents hydrogen, methyl or ethyl,
R² represents hydrogen, methyl or ethyl or one of the groups NR³R⁴, SR⁵ or OR⁵,
R³ and R⁴ in each case represent hydrogen, methyl or ethyl,
R⁵ represents methyl or ethyl, or
R¹ and R³ or R¹ and R⁵ together form a -CH₂-CH₂- group.

4. Process for the preparation of substituted N-methylenethioureas of the general formula (I) 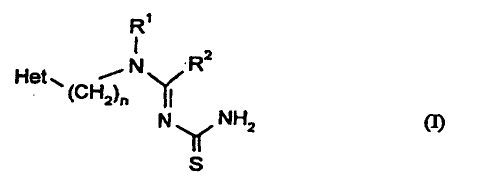 in which
n, Het, R¹ and R² have the meanings given in Claim 1,
**characterized in that** the corresponding N'-aralkyl-N-cyano-amidines of the general formula (II) 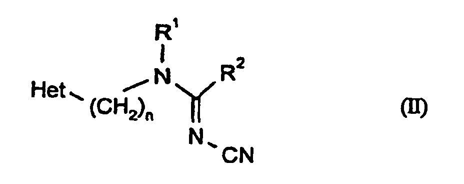 in which
Het, n, R¹ and R² have the abovementioned meaning
are reacted with Lawessons's reagent, if appropriate in the presence of a diluent and if appropriate with the exclusion of moisture.

5. Pesticides, **characterized in that** they comprise at least one N-methylenethiourea of the general formula (I) according to Claim 1.

6. Method of combating animal pests, in particular insects, **characterized in that** N-methylenethioureas of the general formula (I) according to Claim 1 are allowed to act on animal pests and/or their environment.

7. Use of N-methylenethioureas of the general formula (I) according to Claim 1 for combating animal pests, in particular insects.

8. Insecticidal compositions, **characterized in that** they comprise at least one N-methylenethiourea of the general formula (I) according to Claim 1.

9. Process for the preparation of compositions against animal pests, **characterized in that** N-methylenethioureas of the general formula (I) according to Claim 1, are mixed with extenders and/or surfactants.

## Revendications

1. N-méthylènethio-urées substituées de la formule générale (I) 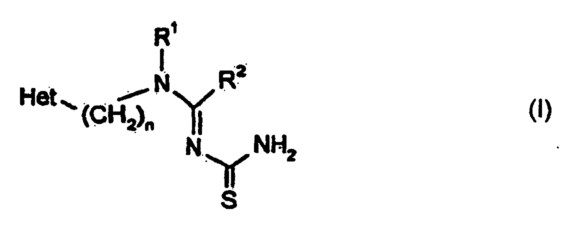 dans laquelle
n représente le nombre 0, 1 ou 2,
Het correspond à un groupement hétérocyclique comportant cinq ou six membres, appartenant à la série constituée des furyle, thiényle, pyrrolyle, pyrazolyle, imidazolyle, 1,2,3- ou 1,2,4-triazolyle, oxazolyle, isoxazolyle, 1,2,4-ou 1,3,4-oxadiazolyle, thiazolyle, isothiazolyle, 1,2,3- 1,2,4- 1,2,5- ou 1,3,4-thiadiazolyle, pyridyle, pyridazinyle, pyrimidinyle et pyrazinyle, portant le(s) substituant(s) suivant(s): fluoro, chloro, bromo, iodo, cyano, nitro, alkyle en C₁-C₄ (qui est substitué le cas échéant par le fluor et/ou le chlore), alcényle en C₂-C₄ (qui est substitué le cas échéant par le fluor et/ou le chlore), alkinyle en C₂-C₄, alcoxyle en C₁-C₄ (qui est substitué le cas échéant par le fluor et/ou le chlore), alcényloxyle en C₃-C₄ (qui est substitué le cas échéant par le fluor et/ou le chlore), alkinyloxyle en C₃-C₄, alkylthio en C₁-C₄ (qui est substitué le cas échéant par le fluor et/ou le chlore), alcénylthio en C₃-C₄ (qui est substitué le cas échéant par le fluor et/ou le chlore), alkinylthio en C₃-C₄, alkylsulfinyle en C₁-C₄ (qui est substitué le cas échéant par le fluor et/ou le chlore), alkylsulfonyle en C₁-C₄ (qui est substitué le cas échéant par le fluor et/ou le chlore), amino, alkylamino en C₁-C₄, di-(alkyle en C₁-C₄)-amino, phényle, phénoxyle, phénylthio, phénylamino, benzyle, formylamino, alkyle en C₁-C₄, carbonylamino, formyle, carbamoyle, (alkyle en C₁-C₄)-carbonyle et/ou (alcoxyle en C₁-C₄)-carbonyle et
R¹ est l'hydrogène ou un alkyle en C₁-C₆,
R² représente l'hydrogène, un alkyle en C₁-C₆ ou un des groupes NR³R⁴, SR⁵ ou OR⁵,
R³ correspond à l'hydrogène ou à un alkyle en C₁-C_{6,}
R⁴ équivaut à l'hydrogène ou à un alkyle en C₁-C_{6,}
R⁵ est un alkyle en C₁-C₆, ou
R¹ et R³ ou R¹ et R⁵ forment ensemble un pont éthylène ou propylène substitué le cas échéant, qui peut être interrompu le cas échéant par un autre atome d'oxygène, d'azote et/ou de soufre et qui peut être substitué le cas échéant par les substituants suivants:
fluoro, chloro, bromo, cyano, alkyle en C₁-C₄, alcoxyle en C₁-C₄, alcényle en C₂-C₄ et alkinyle en C₂-C₄.

2. N-méthylènethio-urées substituées de la formule générale (I) selon la revendication 1, **caractérisées en ce que**
n représente le nombre 0, 1 ou 2,
Het correspond à un groupement hétérocyclique comportant cinq ou six membres, appartenant à la série formée des pyrazolyle, 1,2,4-triazolyle, oxazolyle, isoxazolyle, thiazolyle, isothiazolyle, 1,2,5-thiadiazolyle, pyridyle, pyrazinyle et pirimidinyle, portant le(s) substituant(s) suivant(s): fluoro, chloro, bromo, cyano, nitro, alkyle en C₁-C₂ (qui est substitué le cas échéant par le fluor et/ou le chlore), alcoxyle en C₁-C₂ (qui est substitué le cas échéant par le fluor et/ou le chlore), alkylthio en C₁-C₂ (qui est substitué le cas échéant par le fluor et/ou le chlore) ou alkylsulfonyle en C₁-C₂ (qui est substitué le cas échéant par le fluor et/ou le chlore),
R¹ est l'hydrogène ou un alkyle en C₁-C₄,
R² représente l'hydrogène, un alkyle en C₁-C₄ ou un des groupes NR³R⁴, SR⁵ ou OR⁵,
R³ et R⁴ équivalent chacun à l'hydrogène ou à un alkyle en C₁-C₄,est un alkyle en C₁-C₄, ou R⁵
R¹ et R³ ou R¹ et R⁵ forment ensemble un pont éthylène ou propylène substitué le cas échéant, qui peut être interrompu le cas échéant par un autre atome d'oxygène, d'azote et/ou de soufre et qui peut être substitué le cas échéant par les substituants suivants:
fluoro, chloro, bromo, cyano, méthyle, éthyle, n-, i-propyle, n-, i-, s- ou t-butyle.

3. N-méthylènethio-urées substituées de la formule générale (I) selon la revendication 1, **caractérisées en ce que**
n représente le nombre 1,
Het correspond à un pyridyle substitué par le fluor ou le chlore, ou un thiazolyle substitué par le brome ou le chlore,
R¹ est l'hydrogène, un méthyle ou un éthyle,
R² représente l'hydrogène, un méthyle ou un éthyle ou un des groupes NR³R⁴, SR⁵ ou OR⁵,
R³ et R⁴ équivalent chacun à l'hydrogène, à un méthyle ou à un éthyle,est un méthyle ou un éthyle ou R⁵
R¹ et R³ ou R¹ et R⁵ représentent ensemble un groupe -CH₂-CH₂.

4. Procédé de préparation de N-méthylènethio-urées substituées de la formule générale (I) 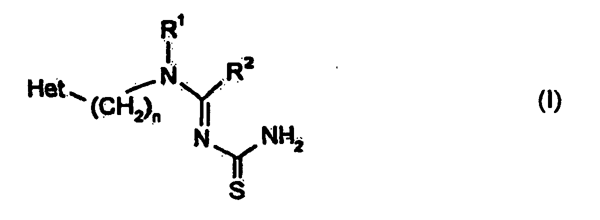 dans laquelle
n, Het, R¹ et R² possèdent la signification mentionnée dans la revendication 1,
**caractérisé en ce que** l'on fait réagir les N'-aralkyl-N-cyanoamidines correspondantes de la formule générale (II) 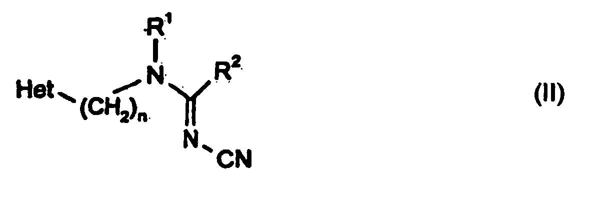 dans laquelle
n, Het, R¹ et R² possèdent la signification mentionnée ci-dessus,
avec un réactif de Lawessons, le cas échéant en présence d'un diluant, le cas échéant sous exclusion d'humidité.

5. Parasiticides, **caractérisés en ce qu'**ils contiennent au moins une N-méthylènethio-urée de la formule générale (I) selon la revendication 1.

6. Procédé de lutte contre les parasites animaux, en particulier les insectes, **caractérisé en ce que** l'on fait agir des N-méthylènethio-urées de la formule générale (I) selon la revendication 1 sur des parasites animaux et/ou sur leur habitat.

7. Utilisation des N-méthylènethio-urées de la formule générale (I) selon la revendication 1 pour combattre les parasites animaux, en particulier les insectes.

8. Insecticides, **caractérisés en ce qu'**ils contiennent au moins une N-méthylènethio-urée de la formule générale (I) selon la revendication 1.

9. Procédé de préparation d'agents destinés à lutter contre les parasites animaux, **caractérisé en ce que** l'on mélange des N-méthylènethio-urées de la formule générale (I) selon la revendication 1 avec des diluants et/ou des agents tensioactifs.
